(19) Europäisches Patentamt
European Patent Office
Office européen des brevets

(11) Veröffentlichungsnummer: **0 103 738**
**A2**

(12) # EUROPÄISCHE PATENTANMELDUNG

(21) Anmeldenummer: 83107949.6

(22) Anmeldetag: 11.08.83

(51) Int. Cl.³: C 08 J 9/08
C 08 L 75/04, C 07 C 69/96

(30) Priorität: 24.08.82 DE 3231397

(43) Veröffentlichungstag der Anmeldung:
28.03.84 Patentblatt 84/13

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Kopp, Richard, Dr.
Wolfskaul 12
D-5000 Köln 80(DE)

(72) Erfinder: Reichmann, Wolfgang, Dr.
Kölner Strasse 48
D-4010 Hilden(DE)

(54) **Verwendung von Carbonylverbindungen und/oder heteroanalogen Carbonylverbindungen als Stabilisierungsmittel für Pyrokohlensäuredialkylester enthaltende Lösungen und, die genannten Verbindungen enthaltende, Polyisocyanat-Zubereitungen.**

(57) Die Verwendung von Carbonylverbindungen und/oder von heteroanalogen Carbonylverbindungen wie gegebenenfalls substituierten Harnstoffen, Thioharnstoff, gegebenenfalls substituierten Sulfamiden, Sulfonsäureamiden, Schwefelsäuredialkylestern, Carbonsäurehalogeniden, Sulfonsäurehalogeniden, Carbonylisocyanaten, Sulfonylisocyanaten oder Carbonsäureanhydriden als Stabilisatoren für, Pyrokohlensäuredialkylester enthaltende, Lösungen in, gegenüber Pyrokohlensäuredialkylestern inerten, organischen Flüssigkeiten, insbesondere in organischen Polyisocyanaten, sowie eine zur Herstellung von Polyurethanschaumstoffen geeignete Polyisocyanatzubereitung, enthaltend

a) mindestens ein flüssiges Polyisocyanat,
b) 0,1 bis 5 Gew.-%, bezogen auf a) mindestens eines Pyrokohlensäuredialkylesters und
c) 10 bis 200 Gew.-%, bezogen auf b) eines Stabilisators der genannten Art.

EP 0 103 738 A2

Croydon Printing Company Ltd

BAYER AKTIENGESELLSCHAFT 5090 Leverkusen, Bayerwerk

Zentralbereich
Patente, Marken und Lizenzen Wr-by-c

# Verwendung von Carbonylverbindungen und/oder heteroanalogen Carbonylverbindungen als Stabilisierungsmittel für Pyrokohlensäuredialkylester enthaltende Lösungen und, die genannten Verbindungen enthaltende, Polyisocyanat-Zubereitungen

Die Erfindung betrifft die Verwendung von bestimmten, nachstehend näher beschriebenen Carbonylverbindungen und/oder von bestimmten nachstehend näher beschriebenen heteroanalogen Carbonylverbindungen als Stabilisierungsmittel für Pyrokohlensäuredialkylester enthaltende Lösungen.

Pyrokohlensäuredialkylester der allgemeinen Formel

$$R{-}O{-}\underset{\underset{O}{\|}}{C}{-}O{-}\underset{\underset{O}{\|}}{C}{-}O{-}R' \qquad (I)$$

für welche

R und R' für gleiche oder verschiedene Alkylreste mit 1 bis 4, vorzugsweise 1 bis 2 Kohlenstoffatome stehen,

insbesondere Pyrokohlensäure-dimethyl- oder -diethylester werden für die unterschiedlichsten Einsatzgebiete eingesetzt. Wegen ihrer bakteriziden und fungiziden Wirksamkeit werden sie z.B. zur Sterilisierung von Getränken und Nahrungsmitteln verwendet (DE-OS 2 223 420, Chem. Abstr. 78, 3020 c, US-PS 3 936 269, Chem. Abstr. 76, 71075 h). In der Veterinärmedizin werden sie wegen ihrer entzündungshemmenden Wirkung und wegen der Beschleunigung der Wundheilung eingesetzt (DE-OS 2 307 827). Ihre leichte Zersetzbarkeit bei Wärmeeinwirkung gegebenenfalls in Gegenwart spezieller Katalysatoren macht man sich beim Einsatz als Treibmittel zur Herstellung geschäumter Kunststoffe (FR-PS 2 220 564, DE-AS 1 694 068, DE-AS 1 694 069 DE-OS 2 053 399) und geschäumter Glasmaterialien (DE-OS 2 153 532, DE-OS 2 318 167) zunutze. Andere Anwendungsgebiete sind z.B. der Einsatz als Aktivator für Peroxidbleichen (DE-OS 2 227 602), als Aktivator bei der peroxidkatalysierten Polymerisation von Vinylchlorid (US-PS 3 584 386, US-PS 3 985 720) als Beschleuniger bei der Polyesterherstellung (US-PS 3 542 738) oder zur Verbesserung des Fließverhaltens von Polyurethan-Systemen (DE-AS 2 524 834).

Die genannten Pyrokohlensäureester weisen die Eigenschaft auf, beim Lagern allmählich und unter Wärmeeinwirkung beschleunigt in Kohlendioxid und das ihnen entsprechende Dialkylcarbonat zu zerfallen (vgl. z.B. V.I. Kovalenko, J. Gen. Chem. USSR, 1952, 22, 1587). Dadurch nimmt die Konzentration von Pyrokchlensäureester enthaltenden Lösungen beim Lagern allmählich ab, und es kann in geschlossenen Gefäßen zu einem nicht unerheblichen Druckaufbau kommen. Es ist deshalb von großem wirtschaftlichem und sicherheitstechnischem Interesse, diesen, insbesondere durch Wärmeeinwirkung bedingten Zerfall der Pyrokohlensäureester zu verringern oder sogar gänzlich zu unterbinden.

Es ist bereits bekannt (Chem. Abstr. 76, 13846 z) zur Verhinderung des thermischen Zerfalls von Pyrokohlensäureestern Borverbindungen, wie z.B. Borsäure, Pyroborsäure oder Bortrioxid zu verwenden. Gemäß einem anderen Vorschlag (Chem. Abstr. 78, 147374 u) wird das gleiche Ziel durch Zugabe von bestimmten Metallsulfaten angestrebt. Gemäß der Lehre dieser Literaturstellen werden die Katalysatoren bei der Herstellung der Pyrokohlensäureester verwendet, wobei man das Rohprodukt in Gegenwart des jeweiligen Stabilisators destilliert, um so ein gereinigtes Produkt in hoher Ausbeute und hoher Reinheit zu erhalten. Die in den genannten Literaturstellen erwähnten Verbindungen weisen jedoch den Nachteil auf, daß sie in den meisten organischen Lösungsmitteln unlöslich sind und somit zur Herstellung von stabilisierten homogenen Lösungen der Pyrokohlensäureester in inerten organischen Flüssigkeiten ungeeignet sind.

Die der Erfindung zugrundeliegende Aufgabe bestand daher darin, wirksame, in organischen Flüssigkeiten lösliche Stabilisatoren für Pyrokohlensäureester bzw. für Pyrokohlensäureester enthaltende Lösungen aufzufinden.

Diese Aufgabe konnte durch die Verwendung der nachstehend näher beschriebenen Carbonylverbindungen bzw. heteroanalogen Carbonylverbindungen gelöst werden.

Gegenstand der Erfindung ist die Verwendung von Carbonylverbindungen und/oder heteroanalogen Carbonylverbindungen,

ausgewählt aus der Gruppe, bestehend aus Harnstoff, N-Alkylharnstoffen, N-Arylharnstoffen, N,N'-Diarylharnstoffen, Thioharnstoff, Sulfamid, N-Arylsulfamiden, N,N'-Diarylsulfamiden, aliphatischen oder aromatischen Sulfonsäureamiden, Schwefelsäuredialkylestern, aliphatischen und aromatischen Carbonsäurehalogeniden, aliphatischen und aromatischen Sulfonsäurehalogeniden, aliphatischen und aromatischen Carbonylisocyanaten, aliphatischen und aromatischen Sulfonylisocyanaten und aliphatischen und aromatischen Carbonsäureanhydriden,

als, die Lagerstabilität erhöhende Zusatzmittel in, Pyrokohlensäuredialkylester enthaltenden, Lösungen in, gegenüber Pyrokohlensäuredialkylestern inerten, organischen Flüssigkeiten.

Die erfindungsgemäße Verwendung eignet sich insbesondere zur Herstellung von stabilisierten, Pyrokohlensäureester enthaltenden Polyisocyanat-Zubereitungen. Gegenstand der Erfindung sind demzufolge auch, zur Herstellung von Polyurethanschaumstoffen geeignete Polyisocyanat-Zubereitungen, enthaltend

a) mindestens ein bei Raumtemperatur flüssiges organisches Polyisocyanat,

b) 0,1 bis 5 Gew.-%, bezogen auf die Komponente a) mindestens eines Pyrokohlensäuredialkylesters der Formel

$$R-O-\overset{\overset{O}{\|}}{C}-O-\overset{\overset{O}{\|}}{C}-O-R'$$

in welcher

R und R' für gleiche oder verschiedene Reste stehen und jeweils Alkylreste mit 1 bis 4 Kohlenstoffatomen bedeuten

und

c) 10 bis 200 Gew.-%, bezogen auf Komponente b) jedoch nicht mehr als 4 Gew.-%, bezogen auf Gesamtgemisch, mindestens einer Carbonylverbindung und/oder mindestens einer heteroanalogen Carbonylverbindung der genannten Art.

Erfindungsgemäß geeignete Stabilisatoren sind

1. Harnstoff;

2. N-Alkylharnstoffe, insbesondere solche mit 1 bis 4 Kohlenstoffatomen im Alkylrest wie z.B. N-Methylharnstoff, N-Ethylharnstoff, N-Isopropylharnstoff oder N-(n-Butyl)-harnstoff;

3. N-Arylharnstoffe, insbesondere solche mit gegebenenfalls inerte Substituenten aufweisenden Arylresten mit insgesamt 6 bis 10 Kohlenstoffatomen wie z.B. N-Phenylharnstoff, N-(p-Tolyl)-harnstoff oder N-Naphthyl-harnstoff;

4. N,N'-Diarylharnstoffe, insbesondere solche mit gegebenenfalls inerte Substituenten aufweisenden Arylresten mit insgesamt 6 bis 10 Kohlenstoffatomen wie z.B. N,N'-Diphenylharnstoff, N,N'-Bis-(p-tolyl)-harnstoff, N-Phenyl-N'-(p-tolyl)-harnstoff oder N-Phenyl-N'-naphthylharnstoff;

5. Thioharnstoff;

6. Sulfamid;

7. N-Arylsulfamide, insbesondere solche mit gegebenenfalls inerte Substituenten aufweisenden Arylresten mit insgesamt 6 bis 10 Kohlenstoffatomen wie z.B.

N-Phenylsulfamid, N-(p-Tolyl)-sulfamid oder N-Naphthyl-sulfamid;

8. N,N'-Diarylsulfamide, insbesondere solche mit, gegebenenfalls inerte Substituenten aufweisenden Arylresten mit insgesamt 6 bis 10 Kohlenstoffatomen wie z.B. N,N'-Diphenylsulfamid, N,N'-Bis-(p-tolyl)-sulfamid oder N,N'-Dinaphthyl-sulfamid;

9. aliphatische oder aromatische Sulfonsäureamide, insbesondere Amide von, gegebenenfalls inerte Substituenten aufweisenden Benzolsulfonsäuren wie z.B. Benzolsulfonsäureamid, p-Toluolsulfonsäureamid, 4-Chlorbenzolsulfonsäureamid, 3-Nitrobenzol-sulfonsäureamid oder 2-Methylbenzolsulfonsäureamid, oder auch Amide von $C_1$-$C_{18}$-Alkansulfonsäuren wie Methan-, Butan- oder Oktodekansulfonsäureamid;

10. Schwefelsäuredialkylester, insbesondere mit Alkylresten mit 1 bis 4, vorzugsweise 1 oder 2 Kohlenstoffatomen wie Dimethylsulfat, Diethylsulfat oder auch Di-(n-butyl)-sulfat;

11. aliphatische oder aromatische Carbonsäurehalogenide, insbesondere -chloride mit 2 bis 10 Kohlenstoffatomen wie Acetylchlorid, n-Propionylchlorid, Benzoylchlorid, Phthaloylchlorid oder Isophthaloylchlorid;

12. aliphatische oder aromatische Sulfonsäurehalogenide, insbesondere -chloride mit 1 bis 10 Kohlenstoffatomen wie Methansulfonsäurechlorid, Butan-1-sulfonsäurechlorid, Benzolsulfonsäurechlorid, 4-Chlorbenzolsulfonsäurechlorid, p-Toluolsulfonsäurechlorid, Benzolsulfonsäurefluorid oder 1,4-Bis-(chlorsulfonyl)-benzol;

13. aliphatische oder aromatische Carbonylisocyanate mit insgesamt 3 bis 10 Kohlenstoffatomen (inklusive den Kohlenstoffatomen der Isocyanatgruppen) wie Acetylisocyanat, Phenylacetylisocyanat, Benzoylisocyanat, p-Nitrobenzoylisocyanat oder Terephthaloyldiisocyanat;

14. aliphatische oder aromatische Sulfonylisocyanate mit insgesamt 2 bis 10 Kohlenstoffatomen (inklusive den Kohlenstoffatomen der Isocyanatgruppen) wie Methansulfonylisocyanat, Benzolsulfonylisocyanat, o- oder p-Toluolsulfonylisocyanat oder o- oder p-Chlorbenzolsulfonylisocyanat;

15. aliphatische oder aromatische Carbonsäureanhydride, insbesondere solche mit insgesamt 4 bis 10 Kohlenstoffatomen wie Essigsäureanhydrid, Propionsäureanhydrid, Bernsteinsäureanhydrid, Glutarsäureanhydrid oder Phthalsäureanhydrid.

Besonders bevorzugte erfindungsgemäß zu verwendende Stabilisatoren sind p-Toluolsulfonylisocyanat und N-Phenylsulfamid.

Die beispielhaft genannten Stabilisatoren werden erfindungsgemäß zur Erhöhung der Lagerstabilität von Pyrokohlensäuredialkylester enthaltenden Lösungen in, gegenüber Pyrokohlensäureestern inerten organischen Flüssigkeiten verwendet.

Bei den Pyrokohlensäureestern handelt es sich um Verbindungen der bereits eingangs genannten Formel, d.h. um Pyrokohlensäuredialkylester mit gleichen oder verschiedenen Alkylresten mit insgesamt 1 bis 4 Kohlenstoffatomen. Bevorzugte Pyrokohlensäuredialkylester im Rahmen der Erfindung sind Pyrokohlensäurediethyl- und -dimethyl-ester.

Bei den, in den erfindungsgemäß zu stabilisierenden Systemen ebenfalls vorliegenden, gegenüber Pyrokohlensäuredialkylestern inerten organischen Flüssigkeiten handelt es sich vorzugsweise um die üblichen organischen Lösungsmittel wie z.B. aliphatische oder aromatische Kohlenwasserstofflösungsmittel, Ester wie z.B. Ethyl- oder Butyl-acetat, Ketone wie z.B. Methyl-ethyl-keton oder Methyl-isobutyl-keton, Etherester wie z.B. Ethylenglykol-monoethyletheracetat, halogenierte Kohlenwasserstofflösungsmittel wie z.B. Trichlorethan. Vorzugsweise handelt es sich bei den, gegenüber Pyrokohlensäureestern inerten organischen Flüssigkeiten jedoch um bei Raumtemperatur flüssige organische Polyisocyanate, wie sie zur Herstellung von Polyurethankunststoffen insbesondere -schaumstoffen Verwendung finden. Beispielhaft genannt seien

2,4- und/oder 2,6-Diisocyanatotoluol, durch Uretanisierung und/oder Carbodiimidisierung verflüssigtes 4,4'-Diisocyanatodiphenylmethan, Hexamethylendiisocyanat, bei Raumtemperatur flüssige, Urethangruppen aufweisende NCO-Präpolymere auf Basis dieser Polyisocyanate und den aus der Polyurethanchemie bekannten Polyhydroxylverbindungen des Molekulargewichtsbereichs 62 bis 6000 und insbesondere bei Raumtemperatur flüssige Polyisocyanatgemische der Diphenylmethanreihe wie sie in bekannter Weise durch Phosgenierung von Anilin/Formaldehyd-Kondensaten zugänglich sind.

Die erfindungsgemäß zu stabilisierenden Lösungen weisen im allgemeinen 0,1 bis 50 Gew.-%, bezogen auf die organische Flüssigkeit, an Pyrokohlensäuredialkylester auf. Die erfindungsgemäß zu stabilisierenden, zur Herstellung von Polyurethanschaumstoffen geeigneten Polyisocyanat-Zubereitungen weisen im allgemeinen 0,1 bis 5, vorzugsweise 0,2 bis 2,0 Gew.-%, bezogen auf die Polyisocyanatkomponente, an Pyrokohlensäuredialkylester auf. Die erfindungswesentlichen Stabilisierungsmittel werden diesen Lösungen in Mengen von mindestens 0,1 Gew.-%, bezogen auf Pyrokohlensäureester, zugesetzt. Im Falle der erfindungsgemäß bevorzugten Stabilisierung der genannten Polyisocyanat-Zubereitungen werden die erfindungswesentlichen Stabilisatoren im allgemeinen in einer Menge von 10 bis 200, vorzugsweise 10 bis 60 Gew.-%, bezogen auf Pyrokohlensäureester, jedoch im allgemeinen maximal in einer Menge von 4 Gew.-%, bezogen auf Gesamtgemisch zugesetzt.

Bei der Herstellung der erfindungsgemäß stabilisierten Lösungen ist die Reihenfolge der Zugabe der Einzelkomponenten zu dem Gesamtsystem selbstverständlich gleichgültig.

Die erfindungsgemäße Stabilisierung bewirkt eine merkliche Erhöhung der Lagerstabilität der, Pyrokohlensäureester enthaltenden, Lösungen. Die erfindungsgemäß stabilisierten Polyisocyanat-Zubereitungen eignen sich insbesondere zur Herstellung von Polyurethanschaumstoffen mit geschlossener Außenhaut gemäß DE-PS 2 524 834 und können anstelle der in dieser Vorveröffentlichung beschriebenen Polyisocyanat-Zubereitungen eingesetzt werden.

In den nachfolgenden Beispielen beziehen sich alle Prozentangaben auf Gew.-%.

Beispiel 1

Jeweils 200 g frisch destilliertes Toluol wurden mit 2 g Pyrokohlensäuredimethylester und 0,6 g eines Stabilisators versetzt. Die Proben wurden dann in einer verschlossenen 250 ml Glasflasche 21 Tage bei 60°C gelagert.
Nach dem Abkühlen wird der Gehalt der einzelnen Proben an Pyrokohlensäuredimethylester gaschromatographisch bestimmt. Die Ergebnisse sind in Tabelle 1 zusammengefaßt.

Tabelle 1

| Stabilisatorzusatz | Restgehalt an Pyrokohlensäuredimethylester nach 21 Tagen/ 60°C (Ausgangskonzentration: 1,0 %) |
|---|---|
| Tosylisocyanat | 1,0 % |
| Harnstoff | 0,5 % |
| Toluolsulfonamid | 0,85 % |
| N-Phenylsulfamid | 1,0 % |
| ohne (Vergleichsprobe) | 0,35 % |

Beispiel 2

Jeweils 200 g eines Polyisocyanatgemischs der Diphenylmethanreihe, hergestellt durch Phosgenierung

eines Anilin/Formaldehyd-Kondensats, welches einen NCO-Gehalt von ca. 31 % und eine Viskosität bei 20°C von 70-150 MPas aufweist wurden mit 2 g Pyrokohlensäuredimethylester und 1 g eines Stabilisators versetzt. Die Proben wurden dann in einer verschlossenen 250 ml Glasflasche 21 Tage bei 60°C gelagert. Nach dem Abkühlen auf Raumtemperatur wurde der Restgehalt an Pyrokohlensäuredimethylester IR-spektroskopisch bestimmt. Hierzu wurden die Proben in eine 0,105 mm dicke Kochsalz-Küvette überführt und das IR-Spektrum an Hand der Pyrokohlensäureester-Absorption bei 1830 $cm^{-1}$ und einer konstanten Isocyanat-Absorption bei 920 $cm^{-1}$ quantitativ ausgewertet. Die Ergebnisse sind in Tabelle 2 zusammengefaßt.

Tabelle 2

| Stabilisatorzusatz | Restgehalt an Pyrokohlensäure-dimethylester nach 21 Tagen/ 60 °C (Ausgangskonzentration: 1,0 %) |
|---|---|
| Harnstoff | 0,68 % |
| N-Phenylharnstoff | 0,65 % |
| N,N'-Diphenylharnstoff | 0,66 % |
| N-Methylharnstoff | 0,58 % |
| Thioharnstoff | 0,55 % |
| N-Phenylsulfamid | 0,71 % |
| N,N'-Diphenylsulfamid | 0,35 % |
| p-Pyrosulfonsäurechlorid | 0,65 % |
| p-Toluolsulfonsäureamid | 0,58 % |
| p-Toluolsulfonylisocyanat | 0,65 % |
| Dimethylsulfat | 0,31 % |
| Terephthaloylchlorid | 0,53 % |
| Acetanhydrid | 0,60 % |
| ohne Stabilisator | 0,18 % |

Beispiel 3

200 g des Polyisocyanatgemischs gemäß Beispiel 2 würden mit 1,7 g Pyrokohlensäuredimethylester und 0,6 g p-Toluolsulfonyl-isocyanat versetzt. Diese Probe und eine Vergleichsprobe, die keinen Stabilisator enthielt, wurden jeweils in einer verschlossenen 250 ml Glasflasche bei 60°C gelagert. Im Wochenabstand wurde der Gehalt an Pyrokohlensäuredimethylester IR-spektroskopisch (siehe Beispiel 2) bestimmt. Tabelle 3 zeigt die gefundenen Werte.

Tabelle 3

| Probe | Restgehalt an Pyrokohlensäuredimethylester in Gew.-% nach | | | | | | | | |
|---|---|---|---|---|---|---|---|---|---|
| | 0 | 1 | 2 | 3 | 4 | 5 | 6 | 7 | 8 |
| | Wochen | | | | | | | | |
| mit Stabilisator | 0,85 | 0,77 | 0,70 | 0,64 | 0,60 | 0,52 | 0,49 | 0,45 | 0,45 |
| ohne Stabilisator | 0,85 | 0,53 | 0,32 | 0,17 | 0,05 | 0 | 0 | 0 | 0 |

Beispiel 4

Jeweils 200 g des Polyisocyanatgemischs gemäß Beispiel 2 wurden mit 2 g Pyrokohlensäuredimethylester und unterschiedlichen Mengen (siehe Tabelle 4) p-Toloylsulfonylisocyanat versetzt und dann in einer verschlossenen

Glasflasche 21 Tage bei 60°C gelagert. Hiernach wurde der Restgehalt an Pyrokohlensäuredimethylester IR-spektroskopisch (siehe Beispiel 2) bestimmt. Tabelle 4 zeigt die gefundenen Ergebnisse:

Tabelle 4

| Zusatzmenge Tosylisocyanat | Restkonzentration an Pyrokohlensäureester (Ausgangskonzentration 1,0 Gew.-%) |
|---|---|
| 0 | <0,05 Gew.-% |
| 0,2 g | 0,32 Gew.-% |
| 0,4 g | 0,63 Gew.-% |
| 0,6 g | 0,66 Gew.-% |
| 1,0 g | 0,64 Gew.-% |

Patentansprüche

1. Verwendung von Carbonylverbindungen und/oder von heteroanalogen Carbonylverbindungen,

ausgewählt aus der Gruppe bestehend aus Harnstoff, N-Alkylharnstoffen, N-Arylharnstoffen, N,N'-Diarylharnstoffen, Thioharnstoff, Sulfamid, N-Arylsulfamiden, N,N'-Diarylsulfamiden, aliphatischen oder aromatischen Sulfonsäureamiden, Schwefelsäuredialkylestern, aliphatischen und aromatischen Carbonsäurehalogeniden, aliphatischen und aromatischen Sulfonsäurehalogeniden, aliphatischen und aromatischen Carbonylisocyanaten, aliphatischen und aromatischen Sulfonylisocyanaten und aliphatischen und aromatischen Carbonsäureanhydriden,

als, die Lagerstabilität erhöhende Zusatzmittel in, Pyrokohlensäuredialkylester enthaltenden, Lösungen in, gegenüber Pyrokohlensäuredialkylestern inerten, organischen Flüssigkeiten.

2. Verwendung von Carbonylverbindungen und/oder heteroanalogen Carbonylverbindungen gemäß Anspruch 1 zur Erhöhung der Lagerstabilität von Lösungen von Pyrokohlensäuredialkylestern in flüssigen organischen Polyisocyanaten.

3. Zur Herstellung von Polyurethanschaumstoffen geeignete Polyisocanat-Zubereitungen, enthaltend

a) mindestens ein bei Raumtemperatur flüssiges organisches Polyisocyanat,

b) 0,1 bis 5 Gew.-%, bezogen auf die Komponente a) mindestens eines Pyrokohlensäuredialkylesters der Formel

$$R{-}O{-}\overset{O}{\overset{\|}{C}}{-}O{-}\overset{O}{\overset{\|}{C}}{-}O{-}R'$$

in welcher

R und R' für gleiche oder verschiedene Reste stehen und jeweils Alkylreste mit 1 bis 4 Kohlenstoffatomen bedeuten

und

c) 10 bis 200 Gew.-%, bezogen auf Komponente b) jedoch nicht mehr als 4 Gew.-%, bezogen auf Gesamtgemisch, mindestens einer Carbonylverbindung und/oder mindestens einer heteroanalogen Carbonylverbindung gemäß Anspruch 1.

4. Polyisocyanat-Zubereitung gemäß Anspruch 3, dadurch gekennzeichnet, daß sie

als Komponente a) ein flüssiges Polyisocyanatgemisch der Diphenylmethanreihe,

als Komponente b) Pyrokohlensäuredimethylester und

als Komponente c) p-Toluolsulfonylisocyanat oder N-Phenylsulfamid enthält.